# EUROPEAN PATENT APPLICATION

(11) **EP 4 071 234 A1**
(43) Date of publication of application: **12.10.2022**
(21) Application number: 21167061.7
(22) Date of filing: 06.04.2021
(51) Int. Cl.: C12M 3/00, C12M 1/12, C12M 1/00, C12N 5/00

(54) **DECIVE FOR FABRICATING A PERFUSABLE THREE-DIMENSIONAL TISSUE CONSTRUCT**

(71) Applicant: Julius-Maximilians-Universität Würzburg, 97070 Würzburg (DE)
(72) Inventor: GROLL, Jürgen, 97246 Eibelstadt (DE); RYMA, Matthias, 97080 Würzburg (DE)
(74) Representative: Vossius & Partner Patentanwälte Rechtsanwälte mbB

(57) **Abstract**

The invention relates to a device for fabricating a perfusable three-dimensional tissue construct as well as to its manufacture and use. The device comprises a chamber in which the tissue construct may be cultivated, the chamber comprising an inlet and an outlet for perfusing the tissue construct, and a sacrificial scaffold fixed in the chamber. The sacrificial scaffold comprises a thermo-responsive polymer, the thermo-responsive polymer being not dissolvable in water at human body temperature and becoming dissolvable in water at a temperature below 30°C, preferably below 25°C. The sacrificial scaffold comprises at least one filament extending from the inlet to the outlet such that dissolution of the sacrificial scaffold provides for a liquid channel from the inlet to the outlet. The sacrificial scaffold seals the inlet and/or the outlet such that dissolution of the sacrificial scaffold after receiving a cultivation matrix in the chamber provides for a liquid channel from the inlet to the outlet through the cultivation matrix.

## Description

### FIELD OF THE INVENTION

The invention relates to specialised perfusion chamber for fabricating perfusable, artificial three-dimensional (3D) tissues. More particularly, it relates to a method, which makes it possible to purposefully fabricate vasculature-like channel networks in perfusion chambers with a fixated thermo-responsive polymer sacrificial scaffold after adding a cell-compatible 3D cultivation matrix (e.g., a hydrogel). Dissolution of the scaffold allows creating, concurrently and/or in one step, the vasculature-like channel network and one or more leakage-free fluid connections for perfusing said network (in particular, by convective liquid transport). The sacrificial scaffold may be arranged in the chamber such that it seals an inlet and/or an outlet of the perfusion chamber, so that it blocks the cultivation matrix from clogging the fluid connections. The one or more connections may be connected to a pump or any other pressure gradient source, such as one or more fluid reservoirs. Dissolution of the scaffold may provide for the fluid connections without requiring further modification of the cultivation matrix, in particular without requiring the creation of holes and/or further channels (e.g., manually) to provide for the one or more fluid connections.

### BACKGROUND OF THE INVENTION

The fabrication of artificial 3D tissue models is an important technology in the fields of basic research, medicine and pharmacy, by which more accurate research results can be achieved compared to conventional 2D cell culture. Due to the more realistic surrounding, cellular interactions in 3D tissue models resemble the situation inside the body more closely. Moreover, artificial 3D tissue constructs in the future may potentially be used for tissue replacement in the human body.

The biggest challenge in the fabrication of artificial 3D tissues is the size limitation of approximately 1 mm, depending on the matrix and cell number, because the cells die due to the insufficient nutrient supply in the interior of the tissues. In the body, the nutrient supply of the entire tissue is ensured through the vascular system, which ramifies from the larger arteries to the micro-vasculature in a network-like manner. Due to their even distribution with a minimum distance of 200 µm between 2 separate vessels, oxygen and nutrients can reach every cell by diffusion. To overcome the size limitation, it is thus important to fabricate microchannels in larger hydrogels in order to keep the cells alive during cultivation. To ensure supply of nutrients to the cells in the artificial 3D tissue through the fabricated channels, a medium with nutrients must be supplied therethrough. In practice, this is accomplished, e.g., by a fluid connection to pump systems, which maintain a flow through the channels via a differential pressure.

The manufacturing and use of reproducible microchannels is known from "Organ-on-a-chip" systems (see US 2014/0302549 A1 and US 10,444,223 B2). In this manner, physiological reactions of organs and organ systems can be emulated by connecting them to a micro-fluidic system, and thereby efficiently supply the cells. Channels of "Organ-on-a-Chip" systems, however, are formed as a portion of a plastic housing on which the cells are applied. These channels, therefore, are not suitable for the manufacturing larger artificial 3D tissues.

Therefore, a matrix is required in which cells can adhere, survive and reproduce in a 3D environment. Hydrogels are particularly suitable for this purpose because the cells can be easily mixed with a liquid initial solution, the so-called precursor solution, and the hydrogel is dimensionally stable after gelation. The initial solution may also be referred to as a crosslinkable composition in the context of the present disclosure.

Various techniques are known for forming channels in artificial tissue models based on hydrogels. These include:
Bioprinting: By additive 3D-printing processes, cells and precursor solutions are printed together directly in a programmed shape. Here, the microchannels are either spared, or a hydrogel component that remains stable over a limited period of time may be used, which can subsequently be dissolved to open the channels. Bioprinting is described in US 2020/0360567 A1. Usual processes include fused deposition modelling (FDM), in-gel-printing and stereolithography (SLA). The main problem of these methods is their low resolution of approximately 0.5 mm, which is based on the low viscosity of the applicable hydrogels and the death rate of the cells due to shear stress induced during printing, especially when using thin printheads to reach high resolutions.

Subsequent dissolution of sacrificial structures (see US 9,242,027 B2): Scaffold structures mainly manufactured by 3D-printing processes are arranged in a precursor solution of a matrix with cells. After crosslinking and subsequent gelation of the solution, the scaffold structures can be removed. This is achieved primarily by dissolution of carbohydrate-based scaffold structures, which dissolve in an aqueous solution.

In order to arrange the sacrificial structures in the hydrogel, they are entirely embedded in the precursor solution. This leads to a full encapsulation of the sacrificial structure. After dissolving the structure, a fluid connection must therefore be fabricated manually because the accesses are blocked by the hydrogel. This is prone to error. Channels with diameters of less than 1 mm have been found to be particularly difficult to hit. Due to this lack of precision and a "breaking" of the hydrogels during subsequent formation of such channels, the connections are also prone to leak.

Moreover, sacrificial structures based on carbohydrates dissolve directly in aqueous solutions. Therefore, time for gelation of the hydrogel may thus be insufficient (depending on the type of mechanism, gelation may require between a minute to several hours). Coating of the sacrificial structures with other materials to provide sufficient time for gelation may thus be required, which is complex, time-consuming and cost-intensive. Also, local hypertension caused by the dissolution of carbohydrates may lead to cells becoming unviable. Furthermore, such sacrificial scaffolds are stable only for a rather short period of time under standard conditions and normal air humidity since they are hygroscopic. Therefore, logistics and storage in economical applications is more laborious.

Aspects of 3D tissue cultivation and corresponding devices are also discussed in Baker et al., Deconstructing the third dimension - how 3D culture microenvironments alter cellular cues, J Cell Sci 125, 3015-3024, doi:10.1242/jcs.079509 (2012); Duval et al., Modeling Physiological Events in 2D vs. 3D Cell Culture, Physiology 32, 266-277, doi: 10.1152/physiol.00036.2016 (2017); Mandrycky et al., Tissue engineering toward organ-specific regeneration and disease modelling, MRS Commun 7, 332-347, doi:10.1557/mrc.2017.58 (2017); Chang et al.. A short discourse on vascular tissue engineering, NPJ Regen Med 2, doi:10.1038/s41536-017-0011-6 (2017); Yang et al., Vascularization in tissue engineering: fundamentals and state-of-art. Progress in Biomedical Engineering 2, 012002, doi: 10.1088/2516-1091/ab5637 (2020); Gelber et al., Model-guided design and characterization of a high-precision 3D printing process for carbohydrate glass, Additive Manufacturing 22, 38-50 (2018); Miller et al., Rapid casting of patterned vascular networks for perfusable engineered three-dimensional tissues, Nature Mater 11, 768-774, https://doi.org/10.1038/nmat3357 (2012); Jingyun et al., Bioprinting of 3D tissues/organs combined with microfluidics, RSC Advances 8, 21712-21727, doi: 10.1039/C8RA03022G (2018); Gryka et al., Controlled dissolution of freeform 3D printed carbohydrate glass scaffolds in hydrogels using a hydrophobic spray coating, Additive Manufacturing 26, 193-201, doi: 10.1016/j.addma.2018.12.014 (2019); Gandhi et al., Studies on thermoresponsive polymers: Phase behaviour, drug delivery and biomedical applications, Asia Journal of Pharmaceutical Sciences 10(2), 99-107 (2015).

It is an objective of the present invention to provide an improved device for fabricating a perfusable three-dimensional tissue construct. In particular, it is an object of the present invention to solve or ameliorate at least some of the drawbacks encountered in the prior art.

### SUMMARY OF THE INVENTION

According to a first aspect of the present disclosure, a device for fabricating a perfusable three-dimensional tissue construct comprises a chamber in which the tissue construct may be cultivated. The chamber comprises at least one inlet and at least one outlet for one or more fluid connections for perfusing the tissue construct. The device also comprises a sacrificial scaffold fixed in the chamber. The sacrificial scaffold comprises a thermo-responsive polymer, which is not dissolvable in water at human body temperature and becomes dissolvable in water at a temperature below 30°C, preferably below 25°C. The sacrificial scaffold comprises at least one filament extending from the inlet to the outlet such that dissolution of the sacrificial scaffold provides for a liquid channel from the inlet to the outlet. The at least one filament may also be referred to as a fiber in the context of the present disclosure.

Preferably, the chamber is configured to receive a tissue cultivation matrix, in which cells of the tissue construct may be cultivated. Dissolution of the sacrificial scaffold after receiving the tissue cultivation matrix in the chamber provides for a liquid channel from the inlet to the outlet through the tissue cultivation matrix. The tissue cultivation matrix may be a crosslinkable composition, in particular a hydrogel.

Preferably, the sacrificial scaffold is arranged in the chamber such that it does at least partially seal and/or is at least partially arranged in the inlet and/or the outlet. More preferably, a portion of the sacrificial scaffold that seals the inlet and/or is arranged in the inlet, and/or a portion of the sacrificial scaffold that seals the outlet and/or is arranged in the outlet is arranged such that it extends out from and/or is not encapsulated in the cultivation matrix when the cultivation matrix is provided in the chamber. In this manner, the inlet and the outlet remain free of cultivation matrix. The liquid channel through the cultivation matrix may thus be created by mere dissolution of the sacrificial scaffold (i.e., simultaneously with dissolution and/or in one step with dissolution), and preferably without a further modification of the cultivation matrix being required (such as creating holes and/or further channels through the cultivation matrix to provide for a liquid connection).

Preferably, the sacrificial scaffold at least partially covers and/or at least partially fills the inlet or the outlet. More preferably, the sacrificial scaffold at least partially covers and/or at least partially fills the inlet and the outlet.

The sacrificial scaffold preferably is fixated to each of the inlet and the outlet. The fixation may be configured to block the inlet and/or the outlet.

The chamber may comprise a bottom and one or more sidewalls. The bottom and/or the sidewalls may be transparent.

The chamber may further comprise a top opening for introducing a cultivation matrix with cells into the chamber (e.g., an initial solution with cells, the initial solution forming a hydrogel). The top opening may be closed via a cover or lid. The cover or lid may be transparent.

Preferably, the inlet is provided in the one or more sidewalls and/or the outlet is provided in the one or more sidewalls. Alternatively, the inlet and/or the outlet could be provided through the cover or lid.

The sacrificial scaffold comprises, or consists of, a thermo-responsive polymer. The thermo-responsive polymer is not dissolvable in water at human body temperature and becomes dissolvable in water at a temperature below 30°C, preferably below 25°C. Human body temperature, as referred to herein, is preferably a temperature in the range of 35 °C to 40 °C, such that the polymer is not dissolvable at any temperature falling within this range. "Not dissolvable" includes in this context that the polymer is a solid at this temperature.

The thermo-responsive polymer may dissolve in water within 30 minutes, preferably within 20 minutes, and more preferably within 15 minutes, at 25°C. Furthermore, the thermo-responsive polymer may have a lower critical solution temperature (LCST) in water in a range of 4°C to 30°C, preferably in a range of 4 °C to 25 °C. The reduction of temperature for a short time period is a very cell friendly and biocompatible stimulus without the need for organic solvents. Furthermore, compared to channel creation via sugar-based fibers, no osmotic pressure is introduced. This may avoid a hypertonic environment and may thus reduce cell death.

The thermo-responsive polymer may have a glass transition temperature (T_{g}) in a range of 30°C to 100°C, preferably 40°C to 100°C, and still more preferably 50°C to 100°C. The glass transition temperature may be determined via differential scanning calorimetry (DSC), e.g. with a heating rate of 20 °C/min. With a high glass transition temperature, sufficient stiffness at room temperature can be ensured. The sacrificial scaffold and/or the device can thus be handled, shipped and/or stored without the requirement of cooling or a special atmosphere.

The thermo-responsive polymer may comprise or consist of repeating units derived from 2-oxazoline or 2-substituted 2-oxazolines as monomers. Preferably, the thermo-responsive polymer is a poly(2-oxazoline) polymer, i.e. a polymer wherein all repeating units are derived from 2-oxazoline or 2-substituted 2-oxazolines as monomers. It is particularly preferred that the thermo-responsive polymer is a homo- or copolymer consisting of repeating units of the formula -N(C(O)R¹)-CH₂-CH₂- which are provided by 2-substituted 2-oxazolines as monomers. R¹ is selected from C1-C6 alkyl and C3-C6 cycloalkyl. For example, it is possible to adjust the LCST of the thermo-responsive polymer via copolymerization of 2-oxazolines carrying different 2-substituents. It is preferably an n-propyl group or a cyclopropyl group, and most preferably a cyclopropyl group. Thus, preferred specific examples of a poly(2-oxazoline) for use as the thermo-responsive polymer in the context of the invention are poly(2-n-propyl-2-oxazoline) and/or poly(2-*cyclo*propyl-2-oxazoline). More preferably, the thermo-responsive polymer comprises, and most preferably consists of, poly(2-*cyclo*propyl-2-oxazoline). With these poly(2-oxazolines), the above-mentioned LCST may be provided. Moreover, a further advantageous feature of, for example, poly(2-*cyclo*propyl-2-oxazoline) is swelling in aqueous solutions. After adding the scaffold to an aqueous solution, the filaments swell because the polymer chains get hydrated. This effect may be employed to abut and/or fuse different structures with each other, thereby allowing to create an interconnected fluid network after dissolution, as discussed further below.

As an alternative example of a thermo-responsive polymer for use in the context of the invention, poly-N-isopropylacrylamide may be mentioned.

Depending on the material used, swelling may lead to deformation of the scaffold if kept in an aqueous solution above 25 °C for longer times, for example for longer than about 15 minutes. It may thus be preferable to employ a fluid initial solution for the cultivation matrix that crosslinks in less than about 15 minutes.

Furthermore, it is preferred that a degree of polymerization n of the thermo-responsive polymer is in a range of 50 to 500, more preferably in a range of 60 to 300, and still more preferably in a range of 70 to 200.

The polymer structure of the preferred poly(2-oxazoline)s as preferred thermo-responsive polymers for use in the context of the invention may thus be illustrated by the following formula (II) 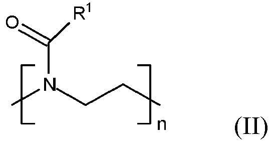 wherein R¹ is a C1-C6 alkyl or a C3-C6 cycloalkyl group, preferably an n-propyl group or a cyclopropyl group, and most preferably a cyclopropyl group, and n is the degree of polymerization, which can be determined, e.g., from the number average molecular weight of the polymer measured by gel permeation chromatography. Preferably, n is in the range of 50 to 500, more preferably 60 to 300, and still more preferably in the range of 70 to 200.

In line with conventional practice, it will be understood that the brackets [ ] in formula (II) indicate that the entity within the brackets (i.e. -N(C(O)R¹)-CH₂-CH₂-) represents a repeating unit of the polymer, and the lines crossing the brackets indicate the bonds which link the repeating unit to adjacent atoms or groups, typically to an adjacent repeating unit or to a terminal group of the polymer.

The 2-substituted 2-oxazoline monomers for the preparation of a poly(2-oxazoline) can be conveniently prepared following procedures known in the art (e.g. H. Witte, W. Seeliger, Justus Liebigs Annalen der Chemie 1974, 6, 996-1009). The poly(2-oxazoline) polymers can be formed e.g. via cationic ring-opening polymerization of 2-oxazoline or 2-substituted 2-oxazolines, respectively. Suitable conditions for the cationic ring opening polymerization and for the work-up of the obtained polymers are also known and are disclosed, e.g., by R. Luxenhofer and R. Jordan, Macromolecules 2006, 39, 3509-3516, or T. Bonne et al., Colloid. Polym. Sci. (2004), 282, 833-843.

The sacrificial scaffold may be configured such that the liquid channel produced has a channel diameter or, for non-round cross sections, minimum width of at least 5 µm, or at least 10 µm along at least some portions thereof. Moreover, the sacrificial scaffold may be configured such that the liquid channel produced has a channel diameter or, for non-round cross sections, maximum width of 500 µm or less, 300 µm or less, 200 µm or less, 50 µm or less, or 25 µm or less along at least some portions thereof. Such diameters and widths are useful for emulating the natural microvasculature. For non-round cross sections, the minimum/maximum width is the smallest/largest width of the channel measured in a cross section of the channel that is taken perpendicular to the direction of the channel's lengthwise extension at the location of the respective cross section.

It is preferred that the sacrificial scaffold comprises one or more filaments. The one or more filaments may be formed by one of 3D-printing, melt electro-writing, solution electrospinning, support bath printing, injection molding (such as micro injection molding) and stereolithography.

The one or more filaments may have a diameter or, for non-round cross sections, width of at least 5 µm, or at least 10 µm. Moreover, the one or more filaments may have a diameter or, for non-round cross sections, width of 500 µm or less, 300 µm or less, 200 µm or less, 50 µm or less, or 25 µm or less. Such diameters and widths are useful for achieving the above-mentioned channel width. For non-round cross sections, the minimum/maximum width is the smallest/largest width of the respective filament measured in a cross section of the filament that is taken perpendicular to the direction of the filament's lengthwise extension at the location of the respective cross section.

It is preferred that the sacrificial scaffold comprises a plurality of connected filaments forming a network, the filaments preferably extending in at least two non-parallel directions along at least a portion thereof.

To create the network, the sacrificial scaffold may have at least one first node at which one filament branches into two or more filaments in a direction from the inlet to the outlet, and/or the sacrificial scaffold may have at least one second node at which two or more filaments merge into one filament in the direction from the inlet to the outlet. More preferably, the scaffold comprises at least 2, at least 3, or at least 5 first and/or second nodes. The number of first nodes may be equal to the number of second nodes.

Alternatively or additionally, the sacrificial scaffold may comprise a plurality of stacked two-dimensional components, each two-dimensional component comprising at least one filament. Each two-dimensional component may comprise a plurality of filaments. The two-dimensional components may be stacked on top of each other.

The two-dimensional components may be connected to form the network. For example, the two-dimensional components may be connected via at least one filament extending in the stacking direction. Swelling of the scaffold may lead to the fusion of stacked or overlapping filaments. This enables the creation of branching interconnected channel networks. Optionally, interconnected channel networks with a single inlet and a single outlet can be created.

For example, two filaments may be fused to create one larger channel portion which branches into two smaller channel portions (e.g., with about half the cross-sectional area than the larger channel section). This allows to emulate the natural microvasculature even further.

A fixation feature may comprise, for example, an adhesive applied to the sacrificial scaffold in the region of the inlet and/or the outlet. Such adhesive may be provided, e.g., by a solution of a thermo-responsive polymer, in particular a thermo-responsive polymer forming the sacrificial scaffold, that is applied and dried. The solution preferably is a solution of the thermo-responsive polymer in either water as a solvent or a mixture of water with an organic solvent. The organic solvent may be ethanol, acetone or another organic solvents. As a preferred example, water or a mixture of ethanol and water may be mentioned, e.g. a mixture comprising 60 to 80 % of ethanol and 20 to 40% water, such as a 70 % ethanol / 30% water mixture, indicated as volume percentage based on the total volume of solvents. Preferably, the solution of the thermo-responsive polymer is a 10 to 50% solution, indicated as the weight percentage of the dissolved polymer based on the total weight of the solution including the solvent and the solute. The solution may be a 10 to 70% solution or a 20 to 60% solution.

Alternatively or additionally, the fixation feature may be formed by a first shape of the sacrificial scaffold that is complementary to a second shape of the chamber in the region of the inlet and/or the outlet. For example, the first shape may be a plug that mates with a second shape formed by a respective socket in the chamber, or vice versa. The plug and/or socket may have a cross section that is larger than a cross section of filaments forming the sacrificial scaffold. For example, the first shape may be formed by a ball-shaped connector wherein the second shape is formed by a cylindrical or spherical socket. Alternatively, the plug may be formed by a filament of the scaffold that is inserted into a respective channel formed by the inlet and/or the outlet. Alternatively, the plug may be provided by an adhesive droplet (e.g., a ball shaped droplet). In this case, the second shape may be configured such that the adhesive may be dripped or injected thereon and/or therein. For example, the second shape may form a structure (e.g., a socket) onto and/or into which the adhesive may be dripped and/or injected. The second shape may be configured to provide for a capillary effect which draws the adhesive into a certain position in which the inlet and/or the outlet are reliably sealed. Such capillary action may draw the adhesive at least partially into the inlet and/or the outlet. The adhesive may be a solution of a thermo-responsive polymer, in particular a thermo-responsive polymer forming the sacrificial scaffold, as described above. The second shape may be configured for having the adhesive dripped (and/or injected) thereon and/or therein after a portion of the sacrificial scaffold is received in the second shape.

The sacrificial scaffold may be attached to a support structure, the support structure being inserted into the chamber.

Preferably, the device is configured such that the inlet and/or the outlet can be easily connected to a system for creating a pressure gradient through the liquid channel formed by dissolution of the sacrificial scaffold.

For this purpose, a first Luer-Lock connector may be connected to the inlet and/or a second Luer-Lock connector may be connected to the outlet. Preferably, the first Luer-Lock connector is connected to the inlet via an inlet channel and/or the second Luer-Lock connector is connected to the outlet via an outlet channel.

Alternatively or additionally, the device may comprise a first liquid compartment in connection to the inlet via an inlet channel and/or a second liquid compartment in connection to the outlet via an outlet channel. Preferably, the first compartment and/or the second compartment is a medium reservoir. The first and or second compartment may have a volume of at least 0,5 ml, preferably at least 1 ml. The device may be configured such that liquid can be flowed from the first compartment to the second compartment and/or vice versa in order to perfuse cells cultivated in the chamber via the liquid channel formed by dissolution of the sacrificial scaffold. This may be achieved, for example, by tilting the device such that the second compartment is arranged below the first compartment to flow liquid from the first compartment to the second compartment and/or by tilting the device in the opposite direction to flow liquid back from the second compartment to the first compartment. In other words, gravity may be used to flow liquid through the liquid channel from the first to the second compartment (and, optionally, vice versa). In this manner, perfusion can be achieved, for example, via a rocking shaker or an orbital shaker.

According to certain embodiments, the device may comprise an upper part defining the first compartment and/or the second compartment, a lower part comprising the sacrificial scaffold, and a separation structure separating the upper part and the lower part. The inlet and/or the outlet of the chamber may then be provided through the separation structure. In particular, the inlet may be fluidly connected to the first compartment and/or the outlet may be fluidly connected to the second compartment. The separation structure may be at least partially formed by a liquid-impermeable membrane, wherein the inlet and/or the outlet of the chamber may be fluidly connected to the first compartment and/or to the second compartment via the liquid-impermeable membrane. Preferably, the device further comprises a partition wall dividing the upper part into the first compartment and the second compartment.

The device may be an insert for a multi-well plate. At least one well of the multi-well plate may form the chamber.

According to a further aspect, the present disclosure relates to a use of the above-mentioned devices. The use comprises providing in the chamber a crosslinkable composition, embedding the sacrificial scaffold in the crosslinkable composition, crosslinking the crosslinkable composition to obtain a crosslinked matrix, cooling the device to dissolve the sacrificial scaffold so that a channel network is formed in the crosslinked matrix as a result of dissolution of the sacrificial scaffold, and perfusing the crosslinked matrix to cultivate the cells. The crosslinkable composition may be a hydrogel. Also, the crosslinkable composition may include living cells.

Crosslinkable compositions which can be suitably provide a crosslinked matrix to incorporate living cells are well known in the art. As exemplary materials which can be used as or which can be comprised by the crosslinkable composition, methacrylated gelatin (GelMA), fibrin gel, agarose gel and alginate may be mentioned. Moreover, hyaluronic acid hydrogels, polymer based hydrogels like polyethylene glycol, or collagen based hydrogels may be used to provide a matrix to incorporate the cells.

According to a further aspect, the present disclosure relates to a method for manufacturing the above-mentioned device. The method comprises (i) forming one or more filaments by melt electro-writing, (ii) providing a body in which the chamber, the first compartment and the second compartment are formed, and (iii) fixing the sacrificial scaffold in the chamber, for example via a form fit and/or adhesion. Alternatively, injection molding or 3D-printing can be used to form one or more filaments.

The method may further comprise forming a plurality of filaments by melt electro-writing and fusing the filaments with each other to obtain the sacrificial scaffold. It is preferred that the filaments are fused with each other by bringing the filaments into contact with an aqueous solution and/or water. For example, after the sacrificial scaffold is obtained, the method may comprise dipping the sacrificial scaffold in the aqueous solution and/or water, and drying the sacrificial scaffold.

As will be appreciated from the above, the present disclosure provides a ready-to-use product that can be conveniently employed to create a perfused 3D cultivation matrix. In particular, a desired hydrogel can be provided in the chamber by adding any kind a crosslinkable initial solution (e.g., with living cells). After crosslinking and reducing the temperature below the LCST (e.g., below 25 °C), the polymer dissolves and, depending on the design of the sacrificial scaffold, leaves a branched liquid channel network, which can be used for delivering nutrients to the cells. Dissolution of the sacrificial scaffold also unseals and/or opens the inlet and/or the outlet (e.g., the inlet channel and/or the outlet channel, respectively). Dissolution of the scaffold thus allows creating, concurrently and/or in one step, the vasculature-like channel network and one or more unblocked leakage-free fluid connections for perfusing said network (in particular, by convective liquid transport).

Dissolution of the sacrificial scaffold can be accelerated by flushing the fully hydrated polymer mass. By providing suitable liquid connectors (e.g., Luer-lock connectors) to the device, the inlet and the outlet can be connected directly to a pump.

The device and method disclosed also conveniently allow to create large networks of liquid channels to perfuse larger tissue constructs. For example, multiple scaffolds may be easily stacked upon and fused with each other. Moreover, the structure of the network and the diameter of the channels can be tailored to the need of different tissues and various spatial requirements, including complex geometries of the chamber.

### BRIEF DESCRIPTION OF THE DRAWINGS

The invention will be described in more detail with reference to the figures below. These figures disclose embodiments of the invention for illustrational purposes only. In particular, the disclosure provided by the figures is not meant to limit the scope of protection conferred by the invention.
Fig. 1a is a schematic cross-sectional side view illustrating an example of a device for fabricating a perfusable, artificial 3D tissue construct according to an embodiment of the present disclosure via a dissolvable scaffold.
Fig. 1b is a schematic cross-sectional side view illustrating the device of Fig. 1a after filling and gelating of a cell-loaded hydrogel in a chamber and dissolving the scaffold.
Fig. 2a is a schematic perspective view showing a device for fabricating a perfusable, artificial tissue construct according to an embodiment of the present disclosure in more detail.
Fig. 2b is a schematic top view of the device of Fig. 2a.
Fig. 2c is a schematic cross-sectional side view of the device of Figs. 2a and 2b.
Fig. 3a shows a schematic flow diagram with a devices according to the present disclosure being connected to a pump.
Fig. 3b shows a schematic flow diagram with several device according to the present disclosure being connected to a pump in parallel.
Fig. 3c shows a schematic flow diagram with several devices according to the present disclosure being connected to a pump in series.
Fig. 4a is a schematic perspective view of a device according to a further embodiment of the present disclosure.
Fig. 4b is a schematic cross-sectional side view showing the device of Fig. 4a.
Fig. 5a is a schematic side view showing a device according to a further embodiment of the present disclosure.
Fig. 5b is a schematic top view showing the device of Fig. 5a.
Fig. 5c shows the device of Figs. 5a and 5b in an open configuration, illustrating an internal volume thereof.
Fig. 5d shows the device of Figs. 5a, 5b and 5c in the open configuration, illustrating a sacrificial scaffold therein.
Fig. 6a shows a schematic cross-sectional side view of a device according to a further embodiment of the present disclosure.
Fig. 6b shows a schematic illustration of a structure forming a first and a second compartment of the device of Fig. 6a.
Fig. 6c shows a schematic illustration of a sacrificial scaffold provided in the device of Fig. 6a.
Fig. 6d shows a schematic illustration of flow through device of Fig. 6a.
Fig. 6e shows the device of Fig. 6a being used in a multiwell plate.

### DETAILED DESCRIPTION

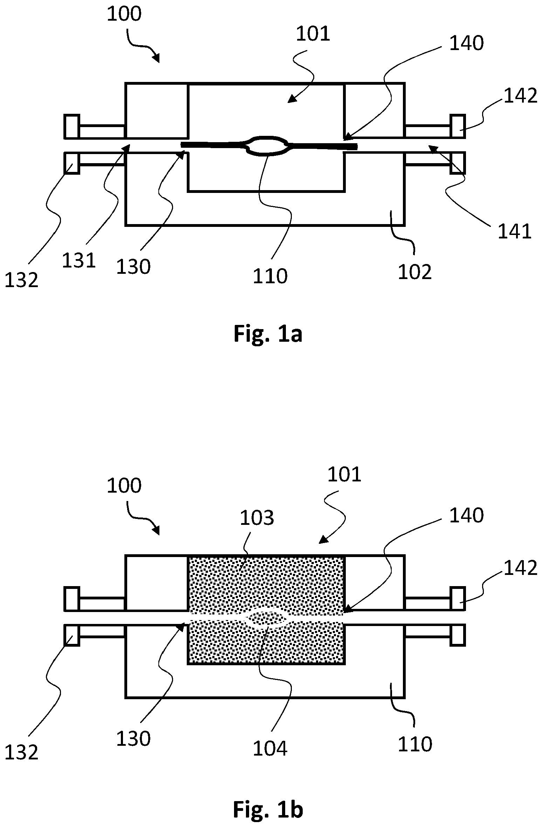

Figs. 1a and 1b are schematic cross-sectional representations illustrating an exemplary device 100 for fabricating a perfusable, artificial 3D tissue construct according to the present disclosure.

Fig. 1a shows the device 100 in its initial configuration. The device 100 comprises a chamber 101 formed in a housing 102. The chamber 101 comprises an inlet 130 connected via an inlet channel 131 to an inlet connector 132 and an outlet 140 connected via an outlet channel 141 to an outlet connector 142. The housing 102 may be formed from any suitable material and by known manufacturing processes. Preferably, the housing 102 is manufactured from a biocompatible material that is not cytotoxic. The housing may be transparent in order to facilitate observation of the 3D tissue structure in a non-invasive manner by conventional microscopy procedures.

A sacrificial scaffold 110 extends from the inlet 130 to the outlet 140 and is fixated in the housing 102. As further shown in Fig. 1a, the sacrificial scaffold may extend into and/or at least partially cover the inlet 130 and the outlet 140.

The scaffold 110 is formed of or comprises one or more polymers that dissolve when appropriate conditions are provided. Preferably, a thermo-responsive polymer that becomes soluble at temperatures below a critical solution temperature, the so-called lower critical solution temperature (LCST), in an aqueous solution is employed. The LCST of the scaffolds is configured to be within the physiological temperature range of 4 to 37°C to avoid temperature-related damages to the cells during dissolution of the scaffold 110. Preferably, the thermo-responsive polymer becomes soluble in an aqueous solution at temperatures below 30°C, for example at temperatures below 25°C. For example, the scaffold 110 may be made from a poly(2-oxazoline) or a poly-N-isopropylacrylamide.

The scaffold 110 may be formed by at least one filament. As shown in Fig. 1a, the filament may comprise at least one node at which a first portion of the scaffold branches into two or more portions. In this manner, the scaffold 110 may be configured to resemble a naturally branching vascular network (i.e., to leave behind such network when dissolved).

The scaffold 110 may be manufactured via, for example, a 3D printing processes, injection moulding or other microfabrication processes for thermoplastic materials. Preferably, melt electrowriting is used, which allows to create a scaffold with very thin filaments. For example, the scaffold may be manufactured by melt electro-writing from poly(2-cyclopropyl-2-oxazoline) to obtain thin filaments having a diameter of less than 100 µm. Such filaments may emulate a micro-vascular network.

The sacrificial scaffold 110 is arranged in the chamber such that it blocks the inlet 130 and/or the outlet 140 so that the cultivation matrix does not clog these fluid connections.

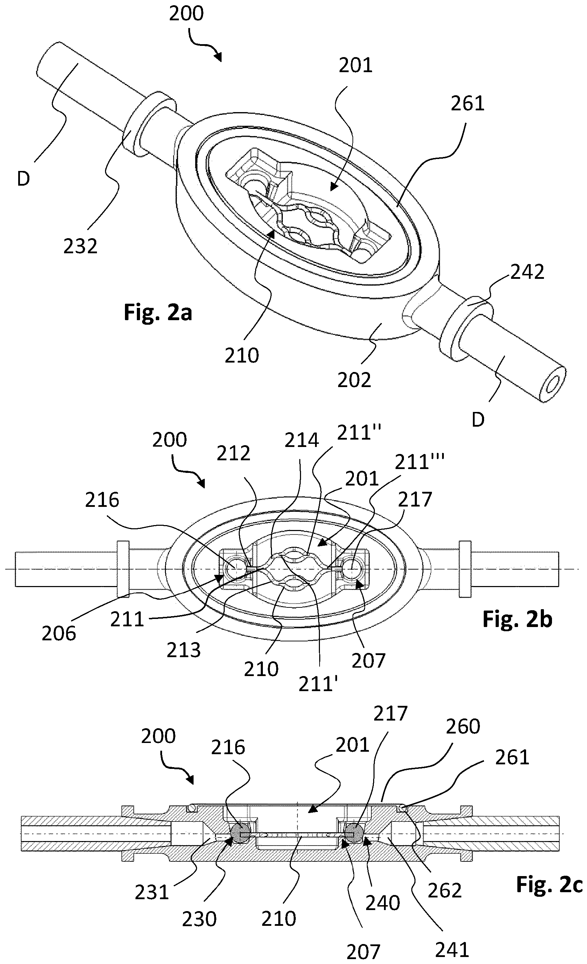

The scaffold 110 may be fixated and sealed with the inlet 130 and/or the outlet 140 by dissolution of the thermo-responsive polymer, melting of the scaffold or other methods. Alternatively or additionally, the sacrificial scaffold 110 may be connected to the inlet 130 and to the outlet 140 via a dissolvable adhesive, and/or correspondingly shaped fixation features may be used (as will be described in relation to Figs. 2a to 2c in more detail below). A suitable adhesive consists, for example, of 50 mass-% of thermo-responsive polymer in pure water. At least 30 mass-% or at least 40 mass-% may also be used.

For example, the scaffold 110 may be produced by a microfabrication process, as mentioned above. The scaffold 110 may then be placed in the chamber 101 and adhered to the inlet 130 and/or to the outlet 140. The adhesive may be dried and the apparatus may subsequently be sterilised.

Fig. 1b shows the device 101 with a tissue cultivation matrix 103 received in the chamber 101. The cultivation matrix 103 may be provided in the chamber 101, for example, by filling a liquid initial precursor solution (not shown) of a hydrogel into the chamber 101 and crosslinking the solution to form the cultivation matrix 103. The initial precursor solution may comprise a plurality of cells that are to be cultivated in the cultivation matrix 103. Once the cultivation matrix 103 has become sufficiently rigid, the scaffold 110 (see Fig. 1a) may be dissolved, thereby leaving one or more liquid channels 104 that extend through the matrix 103 (see Fig. 1b).

For example, the cultivation matrix 103 may be provided by mixing fibroblasts (or other cells) with a methacrylated gelatine precursor solution and lithium phenyl-2,4,6-trimethylbenzoylphosphinate as a crosslinking agent. 0.5 ml of the solution may be filled into the chamber 101. The solution is subsequently crosslinked by ultraviolet light. The polymer dissolves when the temperature decreases to 22°C and leaves behind a network of liquid channels in the gelated methacrylated gelatine through which liquid may be flowed simply by connecting an appropriate supply to the inlet channel 131 and the outlet channel 141 (e.g., via the inlet connector 132 and the outlet connector 142). Subsequently, endothelial cells may be seeded in the channels and cultivated until confluence. Over the entire cultivation period, a medium may be flowed through the channels (e.g., via a pump system) to supply the cells with nutrients from the medium, thus keeping them viable.

Since the scaffold 110 (see Fig. 1a) at least partially or completely seals the inlet 130 and the outlet 140 by extending therein, the scaffold 110 will not be fully encapsulated by the cultivation matrix 103 (i.e., before dissolution). In other words, the inlet 130 and the outlet 140 will remain free of cultivation matrix 103 (they will not be clogged and/or will remain at least partially unobstructed by the crosslinked cultivation matrix 103). Dissolution of the scaffold 110 after crosslinking of the matrix 103 (e.g., gelation of the hydrogel) thus concurrently provides for the fabrication of the one or more liquid channels 104 and a leakage free liquid passage from the inlet 130 to the outlet 140 (and thereby from the inlet connector 132 to the outlet connector 132). Further steps, such as further manual steps for providing a liquid channel through the cultivation matrix 103 may thus be avoided. The liquid channel 104 allowing for the leakage free connection of a nutrient supply (such as a pump and/or one or more reservoirs) may thus be formed in a single step. Problems such as contamination of the matrix and/or leakage of the channels (e.g., at the interface between the matrix and the inlet 130 and/or the outlet 140) may thus be avoided. In particular, the crosslinked matrix 103 may seal around the inlet 130 and the outlet 140.

In addition, the use of a thermo-responsive polymer for the scaffold 110 allows to select a certain trigger temperature at which the scaffold 110 dissolves. Thereby, it may be ensured that the scaffold 110 remains stable for sufficient time in order to allow crosslinking (gelation) of the cultivation matrix 103. Furthermore, the dissolution of such thermo-responsive polymer in water does not generate osmotic pressure, thus avoiding disadvantages associated with, e.g., carbohydrate scaffolds known from the prior art. Scaffolds 110 made from such polymers may also stable and storable for long periods of time under standard conditions and/or normal air humidity.

In order to provide for a convenient connection of the device 100 to a nutrient supply system (e.g., to a system comprising a pump), the inlet connector 132 and/or the outlet connector 142 may be provided with a Luer-lock connector.

As the skilled person will appreciate, the features of the disclosure exemplified with respect to Figs. 1a and 1b above may also be applied in the context of the following examples. This includes, inter alia, the materials and fabrication processes discussed, the general function of the device 100, and the connection to a nutrient supply system (such as by Luer-lock connectors).

Figs. 2a to 2c schematically illustrate a device 200 according to the present disclosure in more detail. Similarly to the device 100 described above, the device 200 comprises a chamber 201 in a housing 202. The device further comprises an inlet connector 232 connected via an inlet channel 231 to an inlet of the chamber 201 and an outlet connector 242 connected via an outlet channel 241 to an outlet of the chamber 201. A sacrificial scaffold 210 is inserted and fixed in the chamber 201.

As shown best in Fig. 2c, the device 200 is further provided with a cover or lid 260 that closes the chamber 201. The cover 260 may be transparent in order to allow for observation of the cells cultivated in the chamber 201. A seal 261 (e.g., an O-ring) may be provided in order to provide a seal between the housing 202 and the cover 260. The seal 261 may extend around the chamber 201 along a groove 262 provided in the housing 202. Such cover may avoid contamination during tissue cultivation. The cover 260 may be configured to enable gas exchange therethrough.

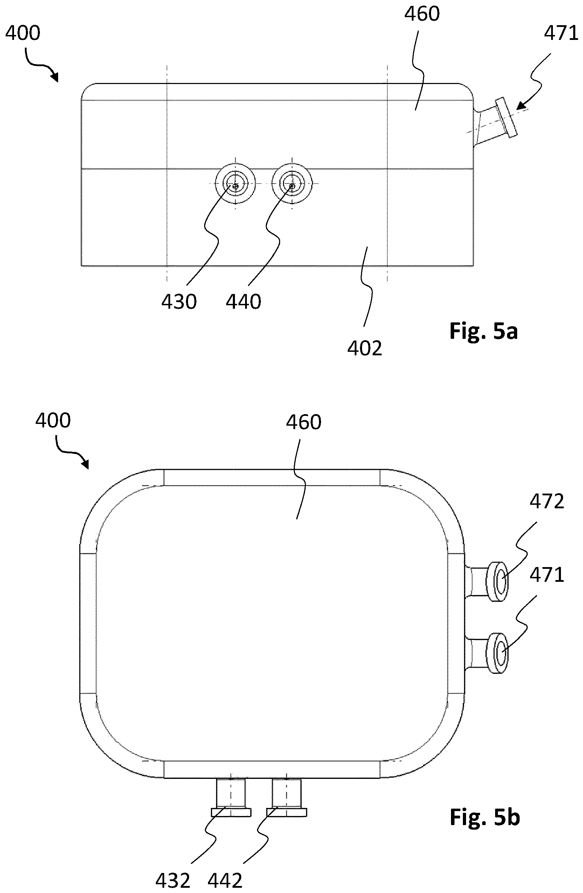

In the device 200 of Figs. 2a to 2c, the cover 260 is opened in order to provide the liquid initial precursor solution of the cultivation matrix in the chamber 201. Alternatively or additionally, one or more additional inlets (and, optionally, also one or more additional outlets) may be provided to the chamber 201 for flowing the initial precursor solution into the chamber 201. Such additional outlets are not shown in Figs. 2a to 2c, but will be discussed in more detail below with reference to Figs. 5a and 5b. The skilled reader will appreciate, however, that such additional one or more inlets (and, optionally, also such additional one or more outlets) may be provided in the device 200 of Figs. 2a and 2b. Therefore, the cover 260 is not necessarily required to be openable.

As further shown in Figs. 2a to 2c, the inlet 230 and the outlet 240 may be connected to respective fluid ducts D for supplying liquid to and draining liquid from the chamber 201 during subsequent cell cultivation. For example, the fluid ducts D may be used for connecting the device 200 to a system providing a pressure gradient, e.g., a pump. While not shown in Figs. 2a to 2c in detail, Luer-lock connectors may be provide for this purpose.

As indicated above, the scaffold may be fixated in the chamber 201, in particular such that the inlet 230 and/or the outlet 240 are covered and/or sealed. In the device 200 of Figs. 2a to 2c, the fixation is provided by providing the scaffold 210 with a first fixation feature 216 that mates with a first engagement feature 206 of the housing 202 and a second fixation feature 217 that mates with a first engagement feature 207 of the housing 202. In particular, the first and second engagement features 206, 207 may be provided in the chamber 201.

The first and second fixation features 216, 217 and the first and second engagement features 206, 207 may be provided with various shapes as long as they are suitably complementary for engaging with each other, preferably in a liquid tight manner to avoid the initial precursor solution to flow into the inlet 230 and/or into the outlet 240. Preferably, the first fixation feature 216 provides a form fit with the first engagement feature 206 and/or the second fixation feature 217 provides a form fit with the second engagement feature 207. As shown in Figs. 2a to 2c, the first and second fixation features 216, 217 may be provided as a male plug (e.g. as a ball-shaped plug) while the first and second engagement features 206, 207 may be provided as a female socket for receiving said plug (e.g., a cylindrical or partially spherical cavity), or vice versa. The ball shaped connectors 216, 217 are substantially larger in cross section than the filaments forming the scaffold 210.

Alternatively, the first and second fixation features 216, 217 may be provided as an adhesive droplet that is dripped and/or injected into a predetermined area (e.g. a cavity) formed in the chamber 201 at 206 and/or 207, respectively (e.g., via a pipette). The cavities formed at 206, 207 may be shaped such that the sacrificial scaffold 210 may be received and/or held therein before dripping the adhesive into the cavities. The respective cavity may be configured to draw the adhesive into the cavity by capillary action, which allows for an adhesive droplet to be placed accurately. In particular, the respective cavity may be configured to draw the adhesive into the inlet 230 and/or into the outlet 240 by capillary action to reliably seal the inlet 230 and/or the outlet 240. The adhesive may be a solution of a thermo-responsive polymer, in particular a thermo-responsive polymer that is also comprised in the sacrificial scaffold.

As already illustrated in Figs. 1a and 1b, but exemplified now in more detail in Figs. 2a and 2b, the scaffold 210 may comprise at least one first node 211 at which at which a first portion 212 of the scaffold 210 branches into two or more second portions 213, 214. As further shown in Fig. 2b, the scaffold may comprise additional nodes 211', 211" and 211"' at which the two or more second portions branch again or the resulting branches merge back into larger portions of the scaffold. Such branching scaffold 210 may be provided in any suitable manner, such as by 3D printing or injection molding (as discussed above). One advantageous form of forming such branching scaffold 210 is by melt electro-writing the filaments forming the scaffold 210. Optionally, the filaments may be fused, e.g. by swelling, at the node 211, 211', 211" and/or 211'".

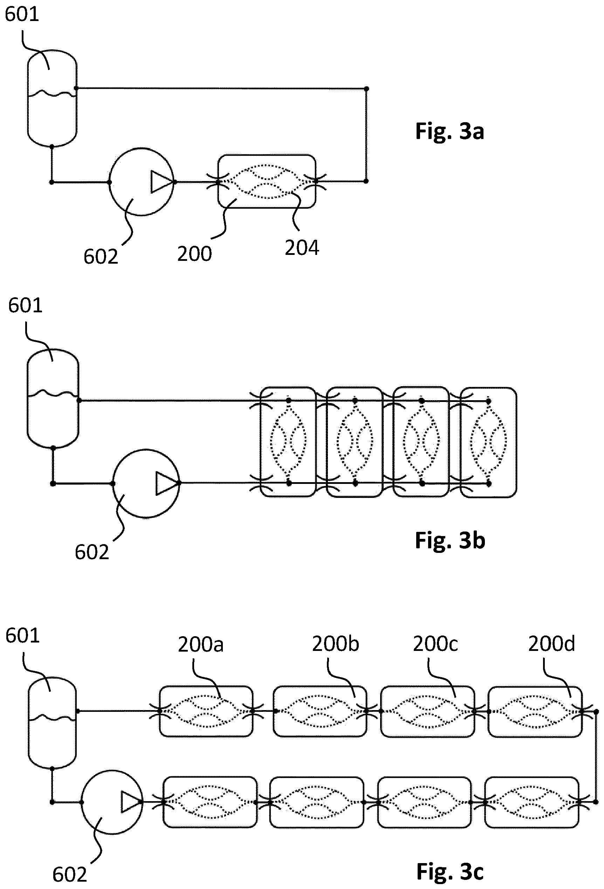

Figs. 3a to 3c provide diagrammatic representations of how devices according to the present disclosure may be connected to a nutrient supply system including a fluid reservoir 601 and a pump 602. While the device 200 according to the foregoing example is schematically shown in these figures, the skilled reader will appreciate that the connection principles shown in Figs. 3a to 3c may be employed in combination with any of the devices disclosed herein.

Fig. 3a shows a single device 200 connected to the fluid reservoir 601 via pump 602. The pump 602 provides for a pressure gradient across the chamber of the device 200 from the inlet to the outlet along the liquid channel 204, thereby providing a flow of fluid through the device 200 and perfusing the 3D tissue construct cultivated in the device's chamber.

Turning now to Figs. 3a and 3b, it can be seen that several devices 200a, 200b, 200c, 200d can be connected in parallel (Fig. 3b) or in series (Fig. 3c) to the fluid reservoir 601 and the pump 602. Those skilled in the art will appreciate that these approaches can also be combined. In this manner, two or more devices (including a large number of devices) can be connected to a single nutrient supply system.

A parallel connection allows, for example, for an efficient and cost-effective cultivation of a plurality of tissue constructs. As shown in Fig. 3a, the devices may be provided with additional fluid connectors to allow for such parallel connection. The respective devices 200 may be configured to be mechanically coupled to each other, e.g. via their respective fluid connectors mating with each other.

A serial connection allows, for example, to cultivate different types of tissue in subsequent devices in order to study their interaction. In at least some of these devices 200, the outlet connector 242 of a first device may be configured to couple directly with the inlet connector 231 of the subsequent device (e.g., by providing corresponding Luer-locks). In this manner, the devices 200 may be connected in a space-efficient manner and without requiring the use of additional tubing. The respective devices 200 may be configured to be mechanically coupled to each other, e.g. via their respective inlets and outlets mating with each other.

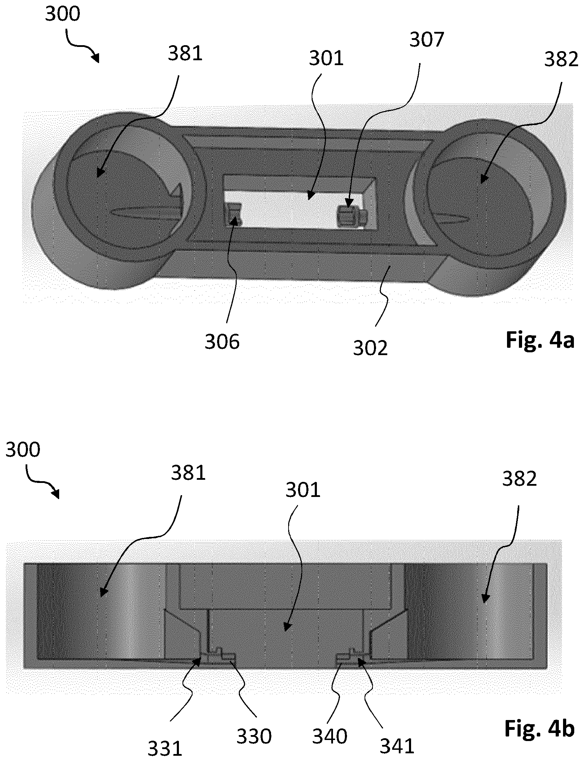

Figs. 4a and 4b show a further example of a device 300 according to the present disclosure. The device 300 comprises a housing 302 in which a chamber 301 is provided. In this case, the chamber 301 is shown without a sacrificial scaffold being provided therein. In the chamber 301, a first engagement structure 306 and a second engagement structure 307 in which the scaffold may be located is provided. When the scaffold is provided therein, it blocks the inlet 330 and/or the outlet 340 of the chamber 301.

The chamber 301 is shown without a bottom surface in Figs. 4a and 4b. The skilled reader will appreciate that such bottom surface may be provided by a separate bottom plate (not shown). Such bottom plate may be transparent. For example, such bottom plate may be provided by a conventional glass slide that is adhered under the chamber in order to facilitate observation of the cell growth during cultivation of the 3D tissue by, e.g., microscopy. The skilled reader will appreciate that any such bottom plate may be attached to the housing 302. Alternatively, a bottom of the chamber 301 may be formed during moulding of the housing 302, i.e. integrally with the housing 302 (also transparent, e.g. by two component injection moulding).

It will be appreciated that a transparent bottom of the chamber (e.g., by providing a transparent plastic or glass in the bottom region of the chamber) may also be provided in conjunction with any of the other devices disclosed herein.

The chamber 301 may be provided with a cover (not shown) as described for the device 200 above. Such cover may avoid contamination during tissue cultivation. It may be configured to enable gas exchange.

The device 300 differs from the device 200 described above in that it comprises a first liquid compartment or reservoir 381 connected to the inlet 330 of the chamber 301 via an inlet channel 341 and a second liquid compartment or reservoir 382 connected to the outlet 340 of the chamber 301 via an outlet channel 341. At least one of the first and second compartments 381, 382 may be filled with a medium comprising nutrients or cells. The medium may then be flowed through the liquid channel formed by dissolution of the sacrificial scaffold by tilting the device 300 from one side to the other and/or by placing the device on a rocking shaker or an orbital shaker.

For example, a rocking shaker generates pressure gradients through a varying inclination, which leads to a bi-directional flow of medium through the tissue construct and so ensures the nutrient supply during cultivation.

In other words, gravity may be used for perfusing the tissue construct cultivated in the chamber 301, thus obviating the need for a more complex supply and/or pumping system.

The first and/or second compartment 381, 382 preferably is formed as an integral part of the housing 302. However, the first and/or second compartments 381, 382 could also be formed as separate elements and connected to the inlet/outlet via a liquid duct (not shown).

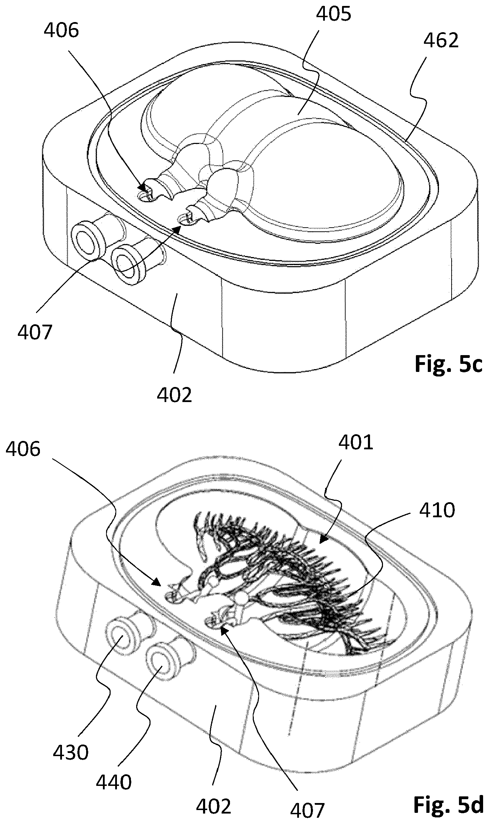

Figs. 5a to 5d show a further example of a device 400 according to the present disclosure. The device 400 comprises a top part or cover 460 and a bottom part or housing 402. The top part 460 and the bottom part 402 together form a chamber 401 (see Fig. 5d) having an inner volume 405. Fig 5c schematically illustrates the device 400 in an open configuration with the top part 460 removed. A possible shape of the inner volume 405 is illustrated. As shown, the inner volume 405 may be provided with complex shapes, e.g. shapes resembling an organ (such as the liver or a kidney).

Optionally, part of the cavity 401 may be formed by and/or extend into the top part 460, as indicated by the inner volume 405 shown in Fig. 5c. The inner volume 405 may thus extend into the top part 460 and/or into the bottom part 402.

The bottom part 402 is provided with at least one inlet 430 and at least one outlet 440 for perfusing a tissue construct cultivated in the device 400. However, the skilled person will appreciate that the inlet 430 and/or the outlet 440 could also be provided in the top part 460, if desired. The inlet, the outlet, or both may be provided with a liquid connector at 432, 442 (e.g., a Luer-lock connector).

As further shown in Figs. 5a and 5b, the top part 460 may be provided with at least one additional inlet 471 for filling a liquid initial precursor solution of a cultivation matrix into the chamber 401. In addition, at least one additional outlet may optionally be provided (e.g., for evacuating air from the chamber 401 when filling the precursor solution therein).

As shown in Fig. 5d, the sacrificial scaffold 410 may be provided with a complex shape and multiple nodes at which filaments forming the scaffold branch from each other or merge back into each other. In other words, by dissolving the scaffold 410 a complex liquid network emulating the natural microvasculature may be provided for perfusing the tissue construct cultivated in the chamber 401. Scaffolds having such complex shapes, including complex 3D shapes, may be manufactured from thermo-responsive polymers, for example, by freeform printing or a similar processes.

As described for previous devices herein, the scaffold 410 may be inserted into the bottom part 402 and may be connected to the inlet 430 and/or to the outlet 440 of the chamber 401 such that dissolution of the scaffold 410 directly creates a liquid network that is coupled to the inlet 430 and/or to the outlet 440, respectively, in a leakage-free manner. Here, only as an example, first and second engagement features 406, 407 are shown into which correspondingly shaped first and second fixation features of the scaffold (only partially visible in Fig. 5d) are engaged. Subsequently, the top part 460 is positioned on the bottom part 402. A seal (not shown) provided in a groove 462 may be used to couple the top and bottom parts 402, 460 in a liquid-tight manner. A cell-loaded hydrogel precursor solution may then be filled through the one or more inlet 472 of the top part 460. These inlets may subsequently be sealed. After gelation of the hydrogel, the scaffold 410 may be dissolved by decreasing the temperature below the LCST. Depending on the shape of the chamber 401 and the scaffold 410 this may result in an organ-like volume of hydrogel provided with a liquid channel network for perfusing the cells. The channel network can be connected directly to, e.g., a pump system in order to perfuse the cultivated cells. After end of the cultivation duration, the mature 3D tissue construct can be removed from the chamber 401.

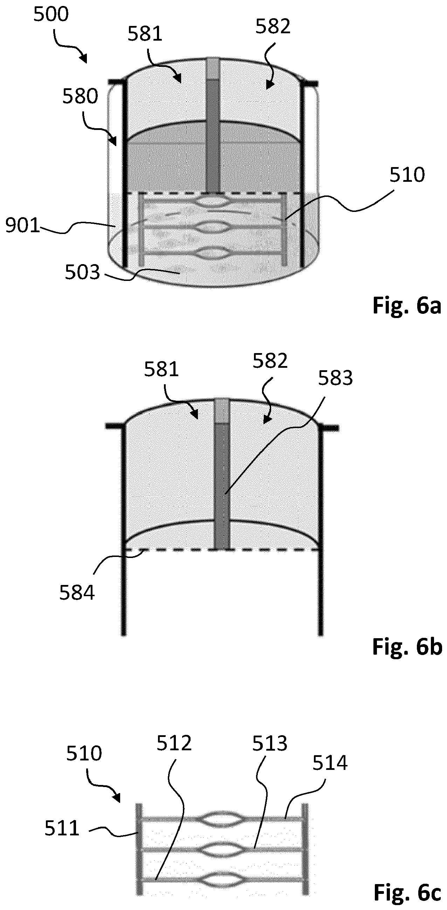

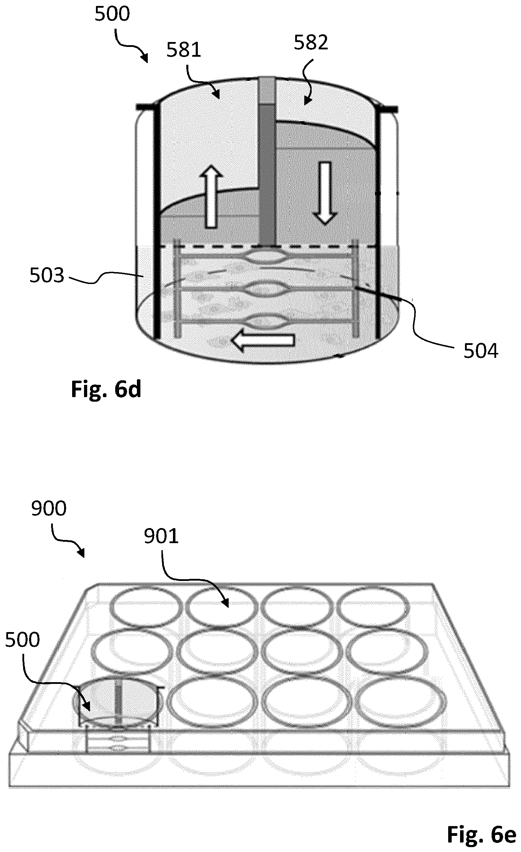

Figs. 6a to 6e schematically illustrate a device 500 according to a further example of the present disclosure. In this example, the device 500 is particularly configured for use in a well 901 of a multiwell plate 900 (see Fig. 6e). The device may be formed as part of the wells 901 of such multiwell plate 900 or may be provided as a separate insert that is inserted into and/or clipped into one or more of the wells 901 of such multiwell plate 900.

As shown in Figs. 6a and 6b, the device 500 may comprise a structure 580 defining a first reservoir or compartment 581 and a second reservoir or compartment 582. The reservoirs or compartments may be delimited by a bottom floor 584, which could be formed by a rigid surface or a membrane that is impermeable to the liquid components of a medium to be provided in the first and/or second reservoirs 581, 582. The first and second reservoirs 581, 582 may be separated from each other by an impermeable membrane or wall 583.

As further shown in Figs. 6a and 6c, a sacrificial scaffold 510 may be provided in the well 901. The scaffold 510 may be connected to the structure 580 below the first and/or second reservoir 581, 582. The scaffold 510 may extend through the bottom floor 584 or be coupled to one or more holes provided through the bottom floor in a liquid-tight manner.

Fig. 6c highlights that the scaffold 510 may be provided with a complex shape to optimize perfusion. For example, the scaffold may include two or more filaments (e.g., three filaments 512, 513, 514) that are stacked on top of each other and/or configured to be located at different depths of the well 901. The filaments 512, 513, 514 may be connected to each other by at least one filament 511 extending in the stacking direction. Each of the stacked filaments 512, 513, 514 may include one or more nodes at which the respective filament branches.

As shown in Figs. 6a, the scaffold 510 may be provided in a cultivation matrix 503 in the well. For example, the structure 580 may be inserted into the well 901 and a liquid initial precursor solution of the cultivation matrix may thereafter be added. Alternatively, the structure 580 with the scaffold 510 attached thereto may be inserted into the well 901 while the precursor solution is still sufficiently liquid and/or soft. The scaffold 510 may be subsequently dissolved by lowering the temperature below the LCST. As illustrated in Fig. 6d, dissolution of the scaffold 510 leaves behind a liquid channel 504 (or channel network) through the cultivation matrix 503. A first end of the channel 504 is connected to the first reservoir 581 and a second end of the channel is connected to the second reservoir 582. A medium may thus be flowed through the channel 504 for perfusing the cells by adding more medium in one of the reservoirs than in the other. Alternatively, the well 901 (or the entire multiwell plate 900) could be tilted.

While the invention has been illustrated and described in detail in the drawings and foregoing description, such illustration and description are to be considered illustrative or exemplary and non-restrictive; the invention is thus not limited to the disclosed embodiments. Variations to the disclosed embodiments can be understood and effected by those skilled in the art and practicing the claimed invention, from a study of the drawings, the disclosure, and the appended claims. In the claims, the word "comprising" does not exclude other elements or steps, and the indefinite article "a" or "an" does not exclude a plurality and may mean "at least one".

The invention may be defined, for example, by the following aspects:
1. A device for fabricating a perfusable three-dimensional tissue construct, comprising:
   a chamber in which the tissue construct may be cultivated, the chamber comprising at least one inlet and at least one outlet for one or more fluid connections for perfusing the tissue construct; and
   a sacrificial scaffold fixed in the chamber;
   wherein the sacrificial scaffold comprises a thermo-responsive polymer, the thermo-responsive polymer being not dissolvable in water at human body temperature and becoming dissolvable in water at a temperature below 30°C, preferably below 25°C;
   wherein the sacrificial scaffold comprises at least one filament extending from the inlet to the outlet such that dissolution of the sacrificial scaffold provides for a liquid channel from the inlet to the outlet.
2. The device of aspect 1, wherein the thermo-responsive polymer dissolves in water within 30 minutes, preferably within 20 minutes, and more preferably within 15 minutes, at 25°C.
3. The device of aspect 1 or 2, wherein the thermo-responsive polymer has a lower critical solution temperature (LCST) in water in a range of 4°C to 30°C.
4. The device of any of the preceding aspects, wherein the thermo-responsive polymer has a glass transition temperature (T_{g}) in a range of 30°C to 100°C.
5. The device of any of the preceding aspects, wherein the thermo-responsive polymer comprises a poly(2-oxazoline), preferably poly(2-n-propyl-2-oxazoline) and/or poly(2-*cyclo*propyl-2-oxazoline), more preferably poly(2-*cyclo*propyl-2-oxazoline).
6. The device of aspect 5, wherein a degree of polymerization n of the thermo-responsive polymer is in a range of 50 to 500.
7. The device of any of the preceding aspects, wherein the sacrificial scaffold is configured to produce a channel having a channel diameter of at least 5 µm, or at least 10 µm.
8. The device of any of the preceding aspects, wherein the sacrificial scaffold is configured to produce a channel having a channel diameter of 500 µm or less, 300 µm or less, 200 µm or less, 50 µm or less, or 25 µm or less.
9. The device of any of the preceding aspects, wherein the sacrificial scaffold comprises one or more filaments.
10. The device of aspect 9, wherein the one or more filaments are formed by one of 3D-printing, melt electro-writing, solution electrospinning, support bath printing, micro injection molding and stereolithography.
11. The device of aspect 9 or 10, wherein the one or more filaments have a diameter of at least 5 µm, or at least 10 µm.
12. The device of aspect 9, 10 or 11, wherein the one or more filaments have a diameter of 400 µm or less, 300 µm or less, 200 µm or less, 50 µm or less, or 25 µm or less.
13. The device of any of aspects 9 to 12, wherein the sacrificial scaffold comprises a plurality of connected filaments forming a network, the filaments preferably extending in at least two non-parallel directions.
14. The device of aspect 13, wherein
   the sacrificial scaffold has at least one first node at which one filament branches into two or more filaments in a direction from the inlet to the outlet, and/or
   the sacrificial scaffold has at least one second node at which two or more filaments merge into one filament in the direction from the inlet to the outlet.
15. The device of aspect 14, comprising at least 2, at least 3, or at least 5 first and/or second nodes.
16. The device of aspect 14 or 15, wherein the number of first nodes is equal to the number of second nodes.
17. The device of any of aspects 9 to 16, wherein the sacrificial scaffold comprises a plurality of stacked two-dimensional components, each two-dimensional component comprising at least one filament.
18. The device of aspect 17, wherein each two-dimensional component comprises a plurality of filaments.
19. The device of aspect 17 or 18, wherein the two-dimensional components are stacked on top of each other.
20. The device of aspect 17, 18, or 19, wherein the two-dimensional components are connected via at least one filament extending in the stacking direction.
21. The device of any of the preceding aspects, wherein
   the sacrificial scaffold at least partially covers and/or at least partially fills the inlet or the outlet, preferably wherein the sacrificial scaffold at least partially covers and/or at least partially fills the inlet and the outlet.
22. The device of any of the preceding aspects, wherein the sacrificial scaffold is attached to a support structure, the support structure being inserted into the chamber.
23. The device of any of the preceding aspects, wherein
   the sacrificial scaffold is fixated to each of the inlet and the outlet by a fixation feature which blocks the inlet and/or the outlet; and
   the fixation feature is formed by applying and drying an adhesive comprising a solution of the thermo-responsive polymer.
24. The device of aspect 23, wherein the solution is a solution of the thermo-responsive polymer in either water or a mixture of water with an organic solvent.
25. The device of aspect 24, wherein the organic solvent is ethanol, acetone or other organic solvents preferably wherein the mixture is a 70% ethanol/water mixture.
26. The device of aspect 24 or 25, wherein the solution of the thermo-responsive polymer is a 10 to 50% solution.
27. The device of any of the preceding aspects, wherein a first Luer-Lock connector is connected to the inlet and/or a second Luer-Lock connector is connected to the outlet, preferably wherein the first Luer-Lock connector is connected to the inlet via a first channel and/or the second Luer-Lock connector is connected to the outlet via a second channel.
28. The device of any of the preceding aspects, further comprising a first compartment in connection to the inlet via a first channel and/or a second compartment in connection to the outlet via a second channel.
29. The device of aspect 28, wherein the first compartment and/or the second compartment is a medium reservoir.
30. The device of any of the preceding aspects, wherein the chamber comprises a bottom and one or more sidewalls.
31. The device of aspect 29, wherein the chamber comprises a top opening for introducing a hydrogel and/or one or more matrices with cells into the chamber.
32. The device of aspect 29 or 30, wherein the inlet is provided in the one or more sidewalls and/or wherein the outlet is provided in the one or more sidewalls.
33. The device of aspect 28 or 29, further comprising
   an upper part defining the first compartment and/or the second compartment;
   a lower part comprising the sacrificial scaffold; and
   a separation structure separating the upper part and the lower part, wherein the inlet and/or the outlet of the chamber are fluidly connected to the first compartment and/or to the second compartment through the separation structure.
34. The device of aspect 33, wherein the separation structure is at least partially formed by a liquid-permeable membrane, wherein the inlet and/or the outlet of the chamber are fluidly connected to the first compartment and/or to the second compartment via the liquid-permeable membrane.
35. The device of aspect 33 or 34, further comprising a partition wall dividing the upper part into the first compartment and the second compartment.
36. The device of aspect 33, 34, or 35, wherein the device is an insert for a multi-well plate.
37. The device of any of aspects 33 to 36, wherein at least one well of the multi-well plate forms the chamber.
38. The device of any of the preceding aspects, wherein the chamber is configured to receive a tissue and/or cell cultivation matrix, in which cells of the tissue construct may be cultivated, wherein dissolution of the sacrificial scaffold after receiving the cultivation matrix in the chamber provides for a liquid channel from the inlet to the outlet through the tissue cultivation matrix.
39. The device of aspect 38, wherein the cultivation matrix is a crosslinkable composition, in particular a hydrogel.
40. Use of the device of any of aspects 1 to 39, comprising:
   providing in the chamber a crosslinkable composition;
   embedding the sacrificial scaffold in the crosslinkable composition;
   crosslinking the crosslinkable composition to obtain a crosslinked matrix;
   cooling the device to dissolve the sacrificial scaffold so that a channel network is formed in the crosslinked matrix as a result of dissolution of the sacrificial scaffold; and
   perfusing the crosslinked matrix to cultivate the cells.
41. The use of aspect 40, wherein the crosslinkable composition is a hydrogel.
42. The use of aspect 40 or 41, wherein the crosslinkable composition includes living cells.
43. A method for manufacturing the device of any of aspects 1 to 39, comprising:
   forming one or more filaments by melt electro-writing (MEW);
   providing a body in which the chamber, the first compartment and the second compartment are formed; and
   fixing the sacrificial scaffold in the chamber.
44. The method of aspect 43, further comprising:
   forming a plurality of filaments by melt electro-writing; and
   fusing the filaments with each other to obtain the sacrificial scaffold.
45. The method of aspect 44, wherein the filaments are fused with each other by bringing the filaments into contact with an aqueous solution and/or water.
46. The method of aspect 45, further comprising, after the sacrificial scaffold is obtained, dipping the sacrificial scaffold in the aqueous solution and/or water; and
   drying the sacrificial scaffold.

## Claims

1. A device for fabricating a perfusable three-dimensional tissue construct, comprising:
a chamber in which the tissue construct may be cultivated, the chamber comprising at least one inlet and at least one outlet for one or more fluid connections for perfusing the tissue construct; and
a sacrificial scaffold fixed in the chamber;
wherein the sacrificial scaffold comprises a thermo-responsive polymer, the thermo-responsive polymer being not dissolvable in water at human body temperature and becoming dissolvable in water at a temperature below 30°C, preferably below 25°C;
wherein the sacrificial scaffold comprises at least one filament extending from the at least one inlet to the at least one outlet such that dissolution of the sacrificial scaffold provides for a liquid channel from the inlet to the outlet;
wherein the sacrificial scaffold seals the inlet and/or the outlet such that dissolution of the sacrificial scaffold after receiving a cultivation matrix in the chamber provides for a liquid channel from the inlet to the outlet through the cultivation matrix.

2. The device of claim 1, wherein the sacrificial scaffold at least partially covers and/or at least partially fills the inlet and/or the outlet.

3. The device of claim 1, wherein the sacrificial scaffold is fixated to the inlet and/or the outlet by a fixation feature that engages with a corresponding engagement feature provided in the chamber, preferably by a form fit.

4. The device of any of the preceding claims, wherein the sacrificial scaffold is fixated to the inlet and/or the outlet by applying and drying an adhesive, preferably wherein the adhesive comprises a solution of a thermo-responsive polymer being not dissolvable in water at human body temperature and becoming dissolvable in water at a temperature below 30°C, preferably below 25°C, more preferably wherein the adhesive comprises the same thermoresponsive polymer than the sacrificial scaffold.

5. The device of any of the preceding claims, wherein the sacrificial scaffold comprises one or more filaments, preferably wherein the sacrificial scaffold comprises a plurality of connected filaments forming a network, the filaments preferably extending in at least two non-parallel directions.

6. The device of any of the preceding claims, wherein
the sacrificial scaffold has at least one first node at which one filament branches into two or more filaments, and/or
the sacrificial scaffold has at least one second node at which two or more filaments merge into one filament.

7. The device of any of the preceding claims, wherein the sacrificial scaffold comprises a plurality of stacked two-dimensional components, each two-dimensional component comprising at least one filament.

8. The device of any of the preceding claims, wherein the chamber comprises a top part with a further inlet for filling a liquid precursor solution of the cell cultivation matrix into the chamber.

9. The device of any of the preceding claims, further comprising a first compartment in connection to the inlet via a first channel and/or a second compartment in connection to the outlet via a second channel, wherein the first compartment and/or the second compartment is a medium reservoir.

10. The device of claim 10, further comprising
an upper part defining the first compartment and/or the second compartment;
a lower part comprising the sacrificial scaffold; and
a separation structure separating the upper part and the lower part, wherein the inlet and/or the outlet of the chamber are fluidly connected to the first compartment and/or to the second compartment through the separation structure.

11. The device of any of the preceding claims, wherein the device comprises a bottom part and a top part, wherein a volume of the chamber configured to receive the cell cultivation matrix therein extends into said bottom part and into said top part.

12. The device of any of the preceding claims, wherein the chamber is configured to receive the cell cultivation matrix, in which cells of the tissue construct may be cultivated, wherein dissolution of the sacrificial scaffold after receiving the cultivation matrix in the chamber provides for the liquid channel from the inlet to the outlet through the cultivation matrix with the cultivation matrix sealing around the inlet and the outlet.

13. The device of any of the preceding claims,
wherein the sacrificial scaffold is configured to produce a channel having a channel diameter of at least 5 µm, or at least 10 µm; and/or
wherein the sacrificial scaffold is configured to produce a channel having a channel diameter of 400 µm or less, 300 µm or less, 200 µm or less, 50 µm or less, or 25 µm or less.

14. Use of the device of any of claims 1 to 13, comprising:
providing in the chamber a crosslinkable composition;
embedding the sacrificial scaffold in the crosslinkable composition;
crosslinking the crosslinkable composition to obtain a crosslinked matrix;
cooling the device to dissolve the sacrificial scaffold so that a channel network is formed in the crosslinked matrix as a result of dissolution of the sacrificial scaffold; and
perfusing the crosslinked matrix to cultivate the cells.

15. A method for manufacturing the device of any of devices 1 to 13, comprising:
forming one or more filaments, preferably by melt electro-writing (MEW), injection moulding or 3D-printing;
providing a body in which the chamber, the first compartment and the second compartment are formed; and
fixing the sacrificial scaffold in the chamber.
